# EUROPEAN PATENT SPECIFICATION

(11) **EP 0 304 292 B1**
(45) Date of publication and mention of the grant of the patent: **02.02.1994**
(21) Application number: 88307634.1
(22) Date of filing: 17.08.1988
(51) Int. Cl.: G01N 33/68

(54) **Antibody to propeptide of procollagen type III, and assay method using it**
Antikörper zum Propeptid des Prokollagen-III und seine Benutzung in einem Analyseverfahren
Anticorps à propeptide de procollagène (III) et procédé d'essai l'utilisant

(30) Priority: 19.08.1987 GB 8719538
(43) Date of publication of application: 22.02.1989
(73) Proprietor: ORION-YHTYMÄ OY, 02101 Espoo (FI)
(72) Inventor: Risteli, Juha, SF 90140 Oulu (FI); Risteli, Leila, SF 90140 Oulu (FI)
(74) Representative: Sexton, Jane Helen

(56) References cited:
- EP-A- 0 004 940
- EP-A- 0 089 008
- EP-A- 0 289 930
- BIOCHEMICAL JOURNAL, vol. 232, no. 1, 1985, London (GB); O. NIEMELA et al., pp. 145-150#
- CLINICAL CHEMISTRY, vol. 31, no. 8, August 1985, Winston, NC (US); O. NIEMELA, pp. 1301-1304#
- CLINICAL CHEMISTRY, vol. 34, no. 4, April 1988, Winston, NC (US); J. RISTELLI et al., pp. 715-718#

## Description

The present invention concerns an improved assay method for the immunological determination of the aminoterminal propeptide of type III procollagen in human serum and preparation of an antiserum suitable for use in this method.

Type III collagen is a collagen type found in several connective tissues throughout the human organism. Its proportion is characteristically high in young tissue, e.g. in the early phases of wound healing or during the initial phase of development of a fibrosis. Many diseases are associated with an increase in the amount of collagen synthesized. If the degradative mechanisms cannot compensate for the increased synthesis, accumulation of collagen in the organ takes place.

Type III collagen is synthesized as a procollagen containing propeptide extensions at both ends of the molecule. The rate of type III collegen synthesis can thus be assessed by determining the amount of propeptide liberated during the conversion of procollagen to collagen. It is known to assay the aminoterminal propeptide of type III procollagen. However, in this assay method small degradation products of the propeptide interfere, making exact measurement of the propeptide impossible and producing practical disadvantages as well as difficulties in the interpretation of the results.

It would, therefore be desirable to solve the problem of interference by the propeptide degradation products and to create a quantitative method, which is quick and simple to practise, for assaying the aminoterminal propeptide of type III procollagen in human serum.

The intact propeptide is in the form of a trimer, whereas the degradation products comprise the globular Col1 domain of the propeptide in monomeric form.

The propeptide is assayed using an antibody, generally in the form of an antiserum, raised against the propeptide. However the antiserum used has an affinity for not only the intact trimeric propeptide, but, to a lesser extent, also for the monomeric form. This is owing to the nature of the propeptide antigen used to raise the antiserum.

The propeptide antigen has been found to contain bacterial collagenase bound to its collagenous Col3 domain after in vitro liberation of the propeptide from procollagen, which is the standard method for its preparation. This Col3 domain can be digested by the enzyme in vitro at a higher temperature than the procollagen proper. Other, naturally occuring proteolytic enzymes are present in the starting material for the alternative isolation procedure (e.g. in ascitic fluid from human cancer patients). Such enzymes are capable of degrading the propeptide in vivo during the immunization process thus inducing antibodies which react with monomeric Col1 degradation products of the propeptide.

The present invention provides trimeric aminoterminal propeptide of type III procollagen free from proteolytic enzymes. Preferably the propeptide is free from enzymes capable of degrading the propeptide to its monomeric form.

The propeptide may be purified from human ascitic fluid as is known to the biochemical experts (Biochemical Journal 1985: 232; 145-150 in which the propeptide was indicated by polyacrylamide slab gel electrophoresis to be a single protein and was analysed using HPLC). The isolated propeptide, which on polyacrylamide slab gel electrophoresis is homogenous and thus biochemically regarded as a single protein, may be finally subjected to low pH and chromatographed on HPLC in acidic condition, preferably less than pH3, to release the bound enzymes applying reverse phase separation. This propeptide is then free from bacterial collagenase.

The present invention also provides an antibody raised against this propeptide, and in particular an antibody which has no affinity for the monomeric form of the propeptide.

This antibody may be raised using techniques known in the art.

The present invention also provides a method of assaying trimeric aminoterminal propeptide of type III procollagen which comprises contacting a sample to be assayed with an antibody of the invention, in the presence of a label, such that the label is bound to the propeptide-antibody complex formed, and assaying the amount of bound and/or unbound label.

The immunoassay of the invention permits the determination of the aminoterminal propeptide of type III procollagen in human serum or other body fluids without serial dilutions. This is due to the fact that the serum samples now give the same slope as the standard antigen and thus the propeptide content of an unknown sample can be directly read from the standard curve. This decreases the amount of tubes needed for the assay to one third and since there are no extra dilution steps the precision of the assay is increased and intraassay and interassay variations less than 5 % can be obtained. The new assay ensures more rapid and accurate analysis of the propeptide concentration especially in those clinical conditions (e.g. the effect of growth hormone treatment in growth hormone deficient children or in non-alcoholic liver disease) where the actual changes are not very large.

The assay may be carried out by contacting with the antibody, labelled aminoterminal propeptide of type III procollagen and the sample to be assayed, separating the propeptide antibody complex so formed from the uncomplexed material and assaying the complexed or uncomplexed label. The propeptide-antibody complex may be contacted with a second antibody which is specific to the first antibody and which may be bound to a solid support. The propeptide-antibody-antibody complex may then be separated from the uncomplexed material.

The immunological assay of the propeptide, using the reagents prepared in the way described, can be carried out e.g. with a radioactive, luminescent, fluorescent or enzymic label either in the antigen or in the antibody. Either polyclonal or monoclonal antibodies can be used. The reference interval for adults is generally 1.7 - 4.2 micrograms/liter serum. In children between 2 years and puberty generally 4.3 - 12.3 micrograms/liter. At puberty there is a significant increase in the serum propeptide level due to increased rate of growth.

The following examples further explain the invention

### EXAMPLE 1

Isolation of the aminoterminal propeptide of human type III procollagen free from proteolytic enzymes.

Five to ten liters of ascitic fluid removed from cancer patients for palliative reasons are precipitated with solid (NH₄)₂SO₄ (40% saturation). The precipitated proteins are collected by centrifugation at 15000 x g for 30 min and dissolved in 50 mM Tris/Hc1, pH 8.6, 2 M urea containing protease inhibitors (3 milligrams/liter of phenylmethylmethylsulfonyl fluoride, N-ethylmaleimide and p-hydroxymercuribenzoate and 10 mM EDTA) and are dialyzed against this buffer. The sample is then chromatographed on a DEAE-Sephacel column (5 x 50 cm) equilibrated in this buffer. Elution is carried out with a linear gradient of NaCl (0-0.4 M NaCl, 4000 + 4000 ml). The propeptide is eluted late in the gradient, clearly after the bulk of other proteins. This material is pooled, dialyzed against 0.2 M NH₄HCO₃, pH 7.9, and lyophilized. The sample is dissolved in 0.2 M NH₄ HCO₃ and chromatographed on a column (1.5 x 110 cm) of Sephacryl S-300 equilibrated in this solution. The fractions containing the propeptide (near the elution position of human IgG) are pooled and dialyzed against 10 mM sodium citrate-HC1 buffer, pH 4.5. They are chromatographed on a column (1.5 x 15 cm) of DEAE-Sephacel equilibrated with this buffer. With a linear gradient of NaCl (0-0.4 M, 500 + 500 ml), the propeptide is eluted between 0.2 and 0.25 M NaCl. The propeptide obtained is homogenous on polyacrylamide slab gel electrophoresis, but still contains proteolytic enzymes attached to it. These can be effectively removed by high performance liquid chromatography applying reverse phase separation in 0.1 % trifluoroacetic acid containing 10 % 2-isopropanol and eluting the bound propeptide with increasing concentrations of 2-isopropanol (10 - 70 %, 0 - 45 min). The propeptide is eluted as a sharp peak in the first half of the gradient.

### EXAMPLE 2

Performance of the equilibrium type of radioimmunoassay:
Ten micrograms of the aminoterminal propeptide of type III procollagen is labelled with 1 millicurie of iodine 125 by chloramine-T (5 micrograms) and the labelled propeptide is separated from free iodine by gel filtration on a Sephacryl S-300 column (1 x 20 cm) equilibrated in PBS-buffer. The labelled propeptide is eluted from the column as a sharp peak well before free iodine. Antiserum binding curves are prepared with 50000 radioactivity counts per minute of the labelled propeptide. The propeptide concentration in an unknown sample of serum or other body fluids is determined in the following radioimmuno inhibition assay: A pretested amount of the antiserum is incubated with the unknown sample and 50 000 counts per minute of the tracer for 2 hours at 37°. Then a solid phase second antibody against rabbit gamma globulin is added and after 15 min incubation at 20° the antigen bound in the immune complex is separated by centrifugation from the solution. The inhibition activity of the unknown sample is compared with the activity of the standard concentrations of unlabelled type III procollagen aminoterminal propeptide.

## Claims (Claims for the following Contracting State(s): AT, BE, CH, DE, FR, GB, GR, IT, LI, LU, NL, SE)

1. Trimeric aminoterminal propeptide of type III procollagen free from proteolytic enzymes.

2. A propeptide as claimed in claim 1 which is free from enzymes capable of degrading the propeptide to its monomeric form.

3. A propeptide as claimed in claim 1 or 2 free from bacterial collagenase.

4. A process for producing a propeptide as claimed in any one of claims 1 to 4 comprising chromatographing the propeptide under acidic conditions applying reverse phase separation.

5. A process according to claim 4 in which the chromatography is carried out at a pH of less than 3.

6. An antibody raised against a propeptide as claimed in claim 1, 2 or 3 or a propeptide produced by a process according to claim 4 or 5.

7. An antibody as claimed in claim 6 which has no affinity for the monomeric form of the propeptide.

8. A method of assaying trimeric aminoterminal propeptide of type III procollagen which comprises contacting a sample to be assayed with an antibody as claimed in claim 6 or 7, in the presence of a label such that the label is bound to the propeptide-antibody complex formed, and assaying the amount of bound and/or unbound label.

9. A method according to claim 8 in which labelled aminoterminal propeptide of type III procollagen and the sample to be assayed are contacted with the antibody, the propeptide-antibody complex so formed is separated from the uncomplexed material and the complexed or uncomplexed label is assayed.

10. A method according to claim 8 or 9 in which the propeptide-antibody complex is contacted with a second antibody specific to the first antibody, and the propeptide-antibody-antibody complex is separated from the uncomplexed material.

11. A method according to claim 10 in which the second antibody is bound to a solid support.

## Claims (Claims for the following Contracting State(s): ES)

1. A process for producing a trimeric aminoterminal propeptide of type III procollagen free from proteolytic enzymes said process comprising chromatographing the propeptide under acidic conditions applying reverse phase separation.

2. A process according to claim 1 in which the propeptide is free from enzymes capable of degrading it to its monomeric form.

3. A process according to claim 1 or 2 in which the propeptide is free from bacterial collagenase.

4. A process according to any one of the preceding claims in which the chromatography is carried out at a pH of less than 3.

5. A method of assaying trimeric aminoterminal propeptide of type III procollagen which comprises contacting a sample to be assayed with an antibody raised against a propeptide produced by a process according to any one of the preceding claims, in the presence of a label such that the label is bound to the propeptide-antibody complex formed, and assaying the amount of bound and/or unbound label.

6. A method according to claim 5 in which labelled aminoterminal propeptide of type III procollagen and the sample to be assayed are contacted with the antibody, the propeptide-antibody complex so formed is separated from the uncomplexed material and the complexed or uncomplexed label is assayed.

7. A method according to claim 5 or 6 in which the propeptide-antibody complex is contacted with a second antibody specific to the first antibody, and the propeptide-antibody-antibody complex is separated from the uncomplexed material.

8. A method according to claim 7 in which the second antibody is bound to a solid support.

## Patentansprüche (Patentansprüche für folgende(n) Vertragsstaat(en): AT, BE, CH, DE, FR, GB, GR, IT, LI, LU, NL, SE)

1. Trimeres aminoterminales Propeptid des Typ III-Procollagens, das frei von proteolytischen Enzymen ist.

2. Propeptid nach Anspruch 1, das frei von Enzymen ist, die zum Abbau des Propeptids in seine monomere Form befähigt sind.

3. Propeptid nach Anspruch 1 oder 2, das frei ist von bakterieller Collagenase.

4. Verfahren zur Herstellung eines Propeptids nach einem der Ansprüche 1 bis 4, umfassend die Chromatographie des Propeptids unter sauren Bedingungen mit Hilfe von Umkehrphasentrennung.

5. Verfahren nach Anspruch 4, wobei die Chromatographie bei einem pH-Wert von weniger als 3 durchgeführt wird.

6. Antikörper, der gegen ein Propeptid nach Anspruch 1, 2 oder 3 erzeugt wurde, oder gegen ein Propeptid, das gemäß einem Verfahren nach Anspruch 4 oder 5 hergestellt wurde.

7. Antikörper nach Anspruch 6, der keine Affinität für die monomere Form des Propeptids besitzt.

8. Verfahren zum Testen des trimeren aminoterminalen Propeptids des Typ III-Procollagens, umfassend das Inkontaktbringen einer zu testenden Probe mit einem Antikörper nach Anspruch 6 oder 7 in Gegenwart eines Markers, so daß der Marker an den gebildeten Propeptid-Antikörper-Komplex gebunden wird, und Bestimmen der Menge an gebundenem und/oder ungebundenem Marker.

9. Verfahren nach Anspruch 8, wobei das markierte aminoterminale Propeptid des Typ III-Procollagens und die zu testende Probe mit dem Antikörper in Kontakt gebracht werden, der so gebildete Propeptid-Antikörper-Komplex vom nicht komplexierten Material abgetrennt wird und der komplexierte und/oder unkomplexierte Marker getestet wird.

10. Verfahren nach Anspruch 8 oder 9, wobei der Propeptid-Antikörper-Komplex mit einem zweiten Antikörper in Kontakt gebracht wird, der spezifisch für den ersten Antikörper ist, und der Propeptid-Antikörper-Antikörper-Komplex vom unkomplexierten Material abgetrennt wird.

11. Verfahren nach Anspruch 10, wobei der zweite Antikörper an einen festen Träger gebunden ist.

## Patentansprüche (Patentansprüche für folgende(n) Vertragsstaat(en): ES)

1. Verfahren zur Herstellung eines trimeren aminoterminalen Propeptids des Typ III-Procollagens, das frei von proteolytischen Enzymen ist, umfassend die Chromatographie des Propeptids unter sauren Bedingungen mit Hilfe von Umkehrphasentrennung.

2. Verfahren nach Anspruch 1, wobei das Propeptid frei von Enzymen ist, die zum Abbau des Propeptids in seine monomere Form befähigt sind.

3. Verfahren nach Anspruch 1 oder 2, wobei das Propeptid frei ist von bakterieller Collagenase.

4. Verfahren nach einem der vorangehenden Ansprüche, wobei die Chromatographie bei einem pH-Wert von weniger als 3 durchgeführt wird.

5. Verfahren zum Testen des trimeren aminoterminalen Propeptids des Typ III-Procollagens, umfassend das Inkontaktbringen einer zu testenden Probe mit einem Antikörper, der gegen ein Propeptid erzeugt wurde, das gemäß einem Verfahren nach einem der vorangehenden Ansprüche hergestellt wurde, in Gegenwart eines Markers, so daß der Marker an den gebildeten Propeptid-Antikörper-Komplex gebunden wird, und Bestimmen der Menge an gebundenem und/oder ungebundenem Marker.

6. Verfahren nach Anspruch 5, wobei das markierte aminoterminale Propeptid des Typ III-Procollagens und die zu testende Probe mit dem Antikörper in Kontakt gebracht werden, der so gebildete Propeptid-Antikörper-Komplex vom nicht komplexierten Material abgetrennt wird und der komplexierte und/oder unkomplexierte Marker getestet wird.

7. Verfahren nach Anspruch 5 oder 6, wobei der Propeptid-Antikörper-Komplex mit einem zweiten Antikörper in Kontakt gebracht wird, der spezifisch für den ersten Antikörper ist, und der Propeptid-Antikörper-Antikörper-Komplex vom unkomplexierten Material abgetrennt wird.

8. Verfahren nach Anspruch 7, wobei der zweite Antikörper an einen festen Träger gebunden ist.

## Revendications (Revendications pour l'(les) Etat(s) contractant(s) suivant(s): AT, BE, CH, DE, FR, GB, GR, IT, LI, LU, NL, SE)

1. Propeptide aminoterminal trimère de procollagène de type III exempt d'enzymes protéolytiques.

2. Propeptide suivant la revendication 1, qui est exempt d'enzymes capables de dégrader le propeptide en sa forme monomère.

3. Propeptide suivant les revendications 1 ou 2, qui est exempt de collagénase bactérienne.

4. Procédé pour produire un propeptide suivant l'une quelconque des revendications 1 à 3, comprenant la chromatographie du propeptide dans des conditions acides avec application d'une séparation en phase inverse.

5. Procédé suivant la revendication 4, dans lequel la chromatographie est effectuée à un pH inférieur à 3.

6. Anticorps dirigé contre un propeptide suivant l'une des revendications 1, 2 ou 3, ou un propeptide produit par un procédé suivant les revendications 4 ou 5.

7. Anticorps suivant la revendication 6, qui n'a aucune affinité pour la forme monomère du propeptide.

8. Procédé d'essai du propeptide aminoterminal trimère de procollagène de type III, qui comprend la mise en contact d'un échantillon à essayer avec un anticorps suivant les revendications 6 ou 7 en présence d'un marqueur de telle sorte que le marqueur est lié au complexe propeptide-anticorps formé, et le dosage de la quantité de marqueur lié et/ou non lié.

9. Procédé suivant la revendication 8, dans lequel le propeptide aminoterminal marqué de procollagène de type III et l'échantillon d'essai sont mis en contact avec l'anticorps, le complexe propeptide-anticorps ainsi formé est séparé de la matière non complexée et le marqueur complexé ou non complexé est dosé.

10. Procédé suivant les revendications 8 ou 9, dans lequel le complexe propeptide-anticorps est mis en contact avec un second anticorps spécifique pour le premier anticorps et le complexe propeptide-anticorps-anticorps est séparé de la matière non complexée.

11. Procédé suivant la revendication 10, dans lequel le second anticorps est lié à un support solide.

## Revendications (Revendications pour l'(les) Etat(s) contractant(s) suivant(s): ES)

1. Procédé pour produire un propeptide aminoterminal trimère de procollagène de type III exempt d'enzymes protéolytiques, qui comprend la chromatographie du propeptide dans des conditions acides avec application d'une séparation en phase inverse.

2. Procédé suivant la revendication 1, dans lequel le propeptide est exempt d'enzymes capables de dégrader le propeptide en sa forme monomère.

3. Procédé suivant les revendications 1 ou 2, dans lequel le propeptide est exempt de collagénase bactérienne.

4. Procédé suivant l'une quelconque des revendications précédentes, dans lequel la chromatographie est effectuée à un pH inférieur à 3.

5. Procédé d'essai du propeptide aminoterminal trimère de procollagène de type III, qui comprend la mise en contact d'un échantillon à essayer avec un anticorps suivant les revendications précédentes en présence d'un marqueur de telle sorte que le marqueur est lié au complexe propeptide-anticorps formé, et le dosage de la quantité de marqueur lié et/ou non lié.

6. Procédé suivant la revendication 5, dans lequel le propeptide aminoterminal marqué de procollagène de type III et l'échantillon d'essai sont mis en contact avec l'anticorps, le complexe propeptide-anticorps ainsi formé est séparé de la matière non complexée et le marqueur complexé ou non complexé est dosé.

7. Procédé suivant les revendications 5 ou 6, dans lequel le complexe propeptide-anticorps est mis en contact avec un second anticorps spécifique pour le premier anticorps et le complexe propeptide-anticorps-anticorps est séparé de la matière non complexée.

8. Procédé suivant la revendication 7, dans lequel le second anticorps est lié à un support solide.
